# EUROPÄISCHE PATENTSCHRIFT

(11) **EP 2 739 203 B1**
(45) Veröffentlichungstag und Bekanntmachung des Hinweises auf die Patenterteilung: **23.11.2016**
(21) Anmeldenummer: 12748665.2
(22) Anmeldetag: 01.08.2012
(51) Int. Cl.: A61C 9/00, A61B 5/00, G06T 7/00

(54) **VERFAHREN ZUR REGISTRIERUNG MEHRERER DREIDIMENSIONALER AUFNAHMEN EINES DENTALEN OBJEKTS**
METHOD FOR REGISTERING A PLURALITY OF THREE-DIMENSIONAL IMAGES OF A DENTAL OBJECT
PROCÉDÉ D'ENREGISTREMENT DE PLUSIEURS VUES EN TROIS DIMENSIONS D'UN OBJET DENTAIRE

(30) Priorität: 01.08.2011 DE 102011080180
(43) Veröffentlichungstag der Anmeldung: 11.06.2014
(73) Patentinhaber: Sirona Dental Systems GmbH, 64625 Bensheim (DE)
(72) Erfinder: KOCHERSCHEIDT, Gerrit, 69190 Walldorf (DE); ADAMSON, Anders, 64293 Darmstadt (DE); POPILKA, Björn, 69502 Hemsbach (DE); THIEL, Frank, 64372 Ober-Ramstadt (DE)
(74) Vertreter: Sommer, Peter
(86) Internationale Anmeldenummer: PCT/EP2012/065011
(87) Internationale Veröffentlichungsnummer: WO 2013/017617

(56) Entgegenhaltungen:
- WO-A2-2009/063087
- US-A1- 2009 231 649
- US-A1- 2010 303 341
- US-B2- 7 698 068
- JACQUET W ET AL: "2D image registration using focused mutual information for application in dentistry", COMPUTERS IN BIOLOGY AND MEDICINE, NEW YORK, NY, US, Bd. 39, Nr. 6, 1. Juni 2009 (2009-06-01), Seiten 545-553, XP026132643, ISSN: 0010-4825, DOI: 10.1016/J.COMPBIOMED.2009.03.011 [gefunden am 2009-05-05]

## Beschreibung

### Technisches Gebiet

Die Erfindung betrifft ein Verfahren zur Registrierung mehrerer dreidimensionaler Aufnahmen eines dentalen Objekts, wobei jede der dreidimensionalen Aufnahmen 3D-Messdaten und Farbdaten einer Messoberfläche des Objekts umfasst, wobei unter Verwendung eines computergestützten Registrierungsalgorithmus die einzelnen Aufnahmen zu einer Gesamtaufnahme zusammengesetzt werden.

### Stand der Technik

Aus dem Stand der Technik sind mehrere Verfahren zur Registrierung dreidimensionaler Aufnahmen bekannt, um diese einzelnen Aufnahmen zu einer Gesamtaufnahme zusammenzusetzen.

Eine Registrierung ist erforderlich, wenn das aufzunehmende Objekt nicht mit einer einzelnen Aufnahme sondern nur mit mehreren Aufnahmen erfasst wird. Dies ist beispielsweise bei räumlich ausgedehnten dentalen Objekten, wie mehrgliedrigen Brücken, oder auch bei Zähnen erforderlich, die von allen Seiten erfasst werden müssen.

US 2009/0231649 A1 offenbart eine Vermessung basierend auf einem holographischen, konoskopischen Vermessungsverfahren, wobei bei der Bearbeitung der aufgenommenen Bilddaten ein Filter verwendet wird, der störende Reflexionen herausfiltert.

WO 2009/063087 A2 offenbart ein Verfahren zur dreidimensionalen optischen Vermessung von dentalen Objekten unter Verwendung eines Triangulationsverfahrens und eines Streifenprojektionsverfahrens.

In US 7,698,068 B2 ist ein Verfahren zur Registrierung überlappender Bereiche offenbart. Im ersten Schritt werden die Datensätze in einen ersten Gewebedatensatz für hartes Gewebe und einen zweiten Gewebedatensatz für weiches Gewebe aufgrund der unterschiedlichen Farbeigenschaften aufgetrennt. In einem zweiten Schritt erfolgt das Zusammenfügen der Datensätze basierend auf den überlappenden Bereichen des ersten Gewebedatensatzes für hartes Gewebe. Somit werden die zweiten Gewebedatensätze für weiches Gewebe bei der Registrierung weggelassen. Dadurch soll die Qualität der zusammengefügten Gesamtaufnahme verbessert werden. Die einzelnen Aufnahmen umfassen 3D-Bilddaten und Farbdaten, die bei einer konfokalen Vermessung des dentalen Objekts erzeugt werden.

Ein Nachteil dieses Verfahrens ist, dass Bereiche mit weichem Gewebe vollständig weggelassen werden und nur Bereiche mit hartem Gewebe zur Registrierung verwendet werden. Dadurch können, insbesondere wenn die Bereiche mit hartem Gewebe klein sind, Registrierungsfehler zu einer fehlerhaften Überlappung der einzelnen Aufnahmen und damit zu einer fehlerhaften Gesamtaufnahme führen.

Die Aufgabe der vorliegenden Erfindung besteht daher darin, ein Verfahren zur Registrierung mehrere dreidimensionaler Aufnahmen eines dentalen Objekts bereitzustellen, das eine möglichst fehlerfreie Registrierung ermöglicht.

### Darstellung der Erfindung

Ein Gegenstand der Erfindung ist ein Verfahren zur Registrierung mehrerer dreidimensionaler Aufnahmen eines dentalen Objekts. Jede der dreidimensionalen Aufnahmen umfasst dabei 3D-Messdaten und Farbdaten einer Messoberfläche des Objekts. Unter Verwendung eines computergestützten Registrierungsalgorithmus werden die einzelnen Aufnahmen zu einer Gesamtaufnahme zusammengesetzt, wobei in jeder der Aufnahmen unter Verwendung eines Segmentierungsverfahrens in Abhängigkeit von den Farbdaten erste Bereiche mit hartem Gewebe und zweite Bereiche mit weichem Gewebe identifiziert werden. Bei der Anwendung des Registrierungsalgorithmus werden die ersten Bereiche und die zweiten Bereiche mit unterschiedlichen Gewichtungsfaktoren gewichtet.

Bei der digitalen Erfassung von dentalen Objekten, insbesondere für CAD/CAM-Systeme, werden mehrere Aufnahmen aus unterschiedlichen Richtungen des dentalen Objekts durchgeführt. Die einzelnen Aufnahmen werden zu der Gesamtaufnahme unter Verwendung des Registrierungsalgorithmus zusammengesetzt. Dabei werden übereinstimmende Strukturen in den einzelnen Aufnahmen erkannt und überlagert. Zur Erkennung der übereinstimmenden Strukturen werden herkömmliche automatisierte Strukturerkennungsverfahren verwendet.

Die einzelnen Aufnahmen können unter Verwendung eines beliebigen dreidimensionalen Aufnahmeverfahrens, wie eines Streifenprojektionsverfahrens oder eines konfokalen Aufnahmeverfahrens, erzeugt sein. Bei einem konfokalen Aufnahmeverfahren, das ein breitbandiges Spektrum verwendet, können die 3D-Messdaten, die die dreidimensionale Struktur des dentalen Objekts wiedergeben, und die Farbdaten, die die tatsächliche Farbe der Objektoberfläche wiedergeben, gleichzeitig ermittelt werden, indem der vom Objekt zurückgestreute Lichtstrahl mittels einer konfokalen optischen Anordnung und mittels eines Farbsensors analysiert wird.

Bei einem Streifenprojektionsverfahren wird das Messobjekt mit einem Muster aus parallelen hellen und dunklen oder auch farbigen Streifen beleuchtet. Diese Streifen entstehen durch Projektion eines optisch strukturierten Elements, z.B. eines Dias oder eines digitalen Lichtmodulators (DMD), in das Messvolumen. In einem weiteren Schritt wird das projizierte Streifenmuster unter einem bekannten Blickwinkel zur Projektion mittels einer digitalen Kamera aufgenommen. Bei der Auswertung wird die Sensorposition eines Streifenübergangs auf dem Kamerasensor exakt einem projizierten Streifen auf dem optisch strukturierten Element zugeordnet. Aus der Sensorposition und in Kenntnis der Streifennummer kann unter Anwendung eines vorbekannten Lochkameramodells für Beobachtungs- und Projektionsstrahlengang die Entfernung des beleuchteten Objekts zu einem vorher festgelegten Ursprung bestimmt werden. Zur exakten Zuordnung der projizierten Streifen können diese entweder farblich kodiert sein oder in ihrer Breite in einem zweiten Schritt zeitlich sequentiell variiert und wieder aufgenommen werden. Bei diesem sogenannten "Graycode"-Verfahren wird somit für jedes Sensorpixel der Kamera eine korrekte Zuordnung zur Lage der projizierten Fläche auf dem optisch strukturierten Element ermittelt und für jedes Pixel kann ein Oberflächenmesspunkt bestimmt werden.

Um die Tiefenauflösung weiter zu erhöhen kann das Streifenprojektionsverfahren, durch ein sogenanntes Phasenschiebeverfahren verbessert werden. In diesem Fall besteht das Muster aus hellen und dunkeln Bereichen nicht aus Streifen mit scharfen Kantenübergängen sondern der Intensitätsverlauf dieses Musters wird sinusoidal moduliert. Die Phasenlage des aufprojizierten Sinusmusters kann sehr genau ermittelt werden, wenn die Phase in Schritten von 90° durchgeschoben wird und von jeder Phasenlage eine Aufnahme gemacht wird. Aus der pixelweise ermittelten Phasenlage kann dann ebenfalls die Raumkoordinate des zugehörigen Oberflächenpunktes ermittelt werden.

Die 3D-Messdaten können mittels eines dentalen Handstücks, das optische Mittel für das Streifenprojektionsverfahrens aufweist, ermittelt werden. Zusätzlich zu diesen Mitteln kann das dentale Handstück eine herkömmliche Farbkamera aufweisen die simultan aus der gleichen Richtung Farbdaten aufnimmt. Die 3D-Messdaten und die Farbdaten können anschließend zu einer Aufnahme zusammengesetzt werden, so dass jedem der Messpunkte der 3D-Messdaten die jeweilige Farbe zugeordnet wird.

Die 3D-Messdaten und die Farbdaten können auch gleichzeitig mittels eines Farbsensor in einem dentalen Handstück vermessen werden. Bei diesen Handstück ist der Einbau einer separaten Farbkamera zur Farbmessung nicht notwendig.

Die 3D-Messdaten und die Farbdaten können auch nacheinander in zwei Aufnahmen vermessen werden. Zwischen den Aufnahmen kann die Beleuchtung umgeschaltet werden. Anschließend werden die beiden Aufnahmen anhand markanter Strukturen oder Marker computergestützt zu einer gemeinsamen Aufnahme zusammengefügt.

Bei den Segmentierungsverfahren werden die ersten Bereiche mit hartem Gewebe und die zweiten Bereiche mit weichem Gewebe unter Verwendung der Farbdaten identifiziert. Beispielsweise kann die Farbe jedes Messpunktes in die Farbton-Komponenten des rot-grün-blau-Farbraums (RGB-Farbraums) oder des HSV-Farbraums zerlegt werden. Anschließend wird jeder Messpunkt unter Anwendung von Schwellwertkriterien einer Gewebeart zugeordnet. Bei hohen Anteilen der roten Farbton-Komponente kann der Messpunkt als weiches Gewebe, nämlich Zahnfleisch, identifiziert werden und bei hohen Anteilen der grünen, blauen und roten Farbtonkomponente kann der Messpunkt als hartes Gewebe, nämlich weiße Zahnsubstanz, identifiziert werden.

Zur Verbesserung der Identifikation des Gewebes kann das Segmentierungsverfahren einen Farbklassifikator verwenden, der mit realen Daten trainiert wird, um den RGB-Farbraum beziehungsweise den HSV-Farbraum entsprechend in die einer Gewebeart zugeordneten Bereiche aufzuteilen. Dabei werden also bestimmte Bereiche im verwendeten Farbraum der jeweiligen Gewebeart zugeordnet. Bereiche, die keiner der beiden Gewebearten zugeordnet werden, werden bei der Registrierung gesondert behandelt. Diese Bereiche können in einem weiteren Schritt einer weiteren ähnlichen Klassifaktion unterzogen, um die Gewebeart mit anderen Reflektionseigenschaften zu identifizieren. Dadurch können auch Bereiche identifiziert werden, die sich im Halbschatten oder an Glanzstellen befinden. Bei einer positiven Identifikation als Zahnersatz oder weiches Gewebe werden diese Bereiche ebenfalls zur Registrierung herangezogen. Darüber hinaus können auch zahnärztliche Hilfsmittel, wie Watte oder Kofferdamm, anhand ihres Farbtons klassifiziert werden, um sie bei der Registrierung gesondert zu gewichten oder wegzulassen. Dadurch wird die Registrierung verbessert.

Durch die Gewichtung der ersten Bereiche, umfassend Zähne oder Zahnersatz, und der zweiten Bereiche, umfassend weiches Gewebe oder zahnärztliche Hilfsmittel, mit unterschiedlichen Gewichtungsfaktoren kann abhängig von der jeweiligen Situation die Qualität der Registrierung verbessert werden. Beispielsweise ist es vorteilhaft bei einem hohen Anteil des harten Gewebes in der Aufnahme das harte Gewebe viel stärker zu gewichten als das weiche Gewebe, da eine Verformung oder Bewegung der Wange oder der Lippe des Patienten zu eine Verformung des weichen Gewebes und damit zu einer fehlerhaften Registrierung führen kann. Falls jedoch der Anteil des harten Gewebes an der gesamten Aufnahme gering ist, kann das weiche Gewebe stärker gewichtet werden, um die Qualität der Registrierung zu verbessern.

Der Vorteil des vorliegenden Verfahrens ist, dass die an die jeweilige Zahnsituation angepasste Gewichtung die Qualität der Registrierung verbessert.

Vorteilhafterweise können die ersten Bereiche Zahnsubstanz oder Zahnersatz und die zweiten Bereiche Zahnfleisch, Zahnfleischersatz oder zahnärztliche Hilfsmittel, wie Watte oder einen Kofferdamm, abbilden.

Der Zahnersatz kann ein beliebiges Zahnersatzteil, wie eine Krone, Inlay, Onlay, Vineer oder eine Teilkrone sein. Der Zahnfleischersatz kann aus ästhetischen Gründen eine ähnliche Farbe wie natürliches Zahnfleisch aufweisen. Zahnärztliche Hilfsmittel können ein breites Spektrum an Farben aufweisen. Zur Verbesserung des Segmentierungsverfahrens können die zahnärztlichen Hilfsmittel möglichst grün oder blau eingefärbt sein, um diese besser vom umgebenden Gewebe zu unterscheiden.

Vorteilhafterweise kann ein erster Flächenanteil der ersten Bereiche an der Gesamtfläche der Aufnahme und ein zweiter Flächenanteil der zweiten Bereiche an der Gesamtfläche der Aufnahme in jeder der einzelnen Aufnahmen berechnet werden. Falls der erste Flächenanteil einen Schwellenwert überschreitet werden nur die ersten Bereiche bei der Anwendung des Registrierungsalgorithmus berücksichtigt. Falls der erste Flächenanteil den Schwellenwert unterschreitet werden die ersten Bereiche mit einem ersten Gewichtungsfaktor und die zweiten Bereiche mit einem zweiten Gewichtungsfaktor gewichtet, wobei der zweite Gewichtungsfaktor bei einem abnehmenden ersten Flächenanteil zunimmt.

Dadurch wird bei einem geringeren ersten Flächenanteil des harten Gewebes das weiche Gewebe stärker gewichtet. Die Registrierung wird dadurch verbessert.

Vorteilhafterweise kann der Schwellenwert für den ersten Flächenanteil auf einen Prozentsatz zwischen 5% und 30% der Gesamtfläche der Aufnahme festgelegt sein.

Dadurch werden die ersten Bereiche mit hartem Gewebe bei der Registrierung oberhalb des Schwellenwerts besonders stark und die zweiten Bereiche mit weichem Gewebe nur schwach oder gar nicht gewichtet. Unterhalb des Schwellenwerts wird das weiche Gewebe verstärkt berücksichtigt. Ein Schwellenwert zwischen 5% und 30% ist vorteilhaft, da die Gewichtung des weichen Gewebes erst bei einem geringen ersten Flächenanteil des harten Gewebes zur Verbesserung der Registrierung führt.

Vorteilhafterweise können die ersten Bereiche mit einem ersten Gewichtungsfaktor von 1,0 gewichtet werden und die zweiten Bereiche mit einem zweiten variablen Gewichtungsfaktor gewichtet werden, der für jeden Messpunkt der Messoberfläche bestimmt wird und bei einem zunehmenden Abstand des Messpunktes zum nächstliegenden ersten Bereich, der hartes Gewebe abbildet, abnimmt. Dies ist eine weitere Alternative der Gewichtung. Durch zunehmende Gewichtung mit dem zunehmenden Abstand zum nächstliegenden ersten Bereich wird sichergestellt, dass in der unmittelbaren Umgebung des ersten Bereichs das weiche Gewebe stärker berücksichtigt wird. Dies berücksichtigt den Umstand, dass Zahnfleisch in Zahnnähe nicht so leicht verformbar ist, wie Zahnfleisch ohne naheliegende Zahnsubstanz und zudem ist die Wahrscheinlichkeit, dass es sich dabei um eine Lippe oder eine Wange handelt gering.

Die Registrierung wird dadurch im Vergleich zu der oben genannten herkömmlichen Registrierung, bei der nur das harte Gewebe berücksichtigt wird, verbessert.

Vorteilhafterweise kann der zweite Gewichtsfaktor an der Grenze zum ersten Bereich einen Wert von 1,0 betragen und mit zunehmendem Abstand zum nächstliegenden ersten Bereich bis zu einem minimalen Wert von 0,0 abnehmen.

Dadurch wird das weiche Gewebe an der Grenze zum harten Gewebe mehr berücksichtigt und die Zuverlässigkeit der Registrierung erhöht.

Vorteilhafterweise können mithilfe von Schwellenwertkriterien, die auf die Farbdaten angewendet werden, dritte Bereiche identifiziert werden, die weder den ersten Bereichen mit harten Gewebe noch den zweiten Bereiche mit weichem Gewebe zugeordnet werden können. Die dritten Bereiche können beispielsweise zu helle Bereiche, die aufgrund von Glanzlicht entstehen, oder zu dunkle Bereiche, die aufgrund einer unzureichenden Beleuchtung entstehen, sein. Für die dritten Bereiche wird ein dritter Flächenanteil an der Gesamtfläche der Aufnahme berechnet.

Dieser dritte Flächenanteil stellt ein Qualitätskriterium für die Aufnahmen dar. Falls der dritte Flächenanteil zu groß ist und beispielsweise einen Prozentsatz von 40 % überschreitet ist die jeweilige Aufnahme unbrauchbar und muss wiederholt werden. Erst wenn der dritte Flächenanteil klein genug ist kann die jeweilige Aufnahme für die Registrierung verwendet werden. Dabei werden die dritten Bereiche nicht berücksichtigt beziehungsweise mit einem Gewichtungsfaktor von 0,0 gewichtet.

Dadurch werden fehlerhafte Stellen, die durch Glanzlicht oder unzureichende Beleuchtung erzeugt wurden, weggelassen, so dass dadurch die Registrierung verbessert wird. Denn die fehlerhaften Bereiche, die durch Glanzlicht oder fehlerhafte Beleuchtung verursacht wurden, erscheinen abhängig von der Aufnahmerichtung und der Einstrahlrichtung der Beleuchtung in jeder der Aufnahmen an unterschiedlichen Stellen.

Vorteilhafterweise kann vor der Segmentierung in erste Bereiche und zweite Bereiche ein Blur-Filter auf die Farbdaten in jede der Aufnahmen angewendet werden.

Dadurch wird die Aufnahme geglättet und die Farbsegmetierung führt inbesondere an Übergängen zwischen den ersten Bereichen und den zweiten Bereichen zu besseren Ergebnissen, da diese Übergängen an denen die Unterscheidung geringfügig ist auf Grund von elektronischem Rauschen der Kamera ausgefranst wirken. Diese Ausfransungen werden somit geglättet, so dass die berechneten Übergänge zwischen den ersten Bereichen und den zweiten Bereichen die tatsächlichen Übergänge in der realen Situation im Mund des Patienten besser wiedergeben.

Vorteilhafterweise kann jede Aufnahme mittels eines dentalen Handstücks aufgenommen werden, das erste Mittel zur Erzeugung der 3D-Messdaten und zweite Mittel zur Erzeugung der Farbdaten umfasst, wobei die 3D-Messdaten und die Farbdaten gleichzeitig oder in einem kurzen Zeitabstand aus der gleichen Aufnahmerichtung erfasst werden.

Dadurch können sowohl die 3D-Messdaten als auch die Farbdaten gleichzeitig mittels des dentalen Handstücks aufgenommen werden. Mittels eines Computers können dann die 3D-Messdaten mit den Farbdaten zusammengesetzt werden und jedem der Messpunkte in den 3D-Messdaten der jeweilige Farbwert zugeordnet werden.

Vorteilhafterweise können die 3D-Messdaten unter Verwendung der ersten Mittel, die auf einem Triangulationsverfahrens und einem Streifenprojektionsverfahrens beruhen, und die Farbdaten unter Verwendung einer herkömmlichen Farbkamera erzeugt werden.

Dadurch umfasst das dentale Handstück sowohl die ersten Mittel für das Streifenprojektionsverfahren als auch die Farbkamera für die Ermittlung der Farbdaten. Die 3D-Messdaten und die Farbdaten werden somit aus der gleichen Aufnahmerichtung gleichzeitig erfasst.

Vorteilhafterweise kann der Zeitabstand zwischen 0,2 ms und 100 ms betragen.

Dadurch ist der Zeitabstand kurz genug, um eine Verwacklung zwischen der Aufnahme der 3D-Messdaten und der Aufnahme der Farbdaten zu vermeiden, die durch eine typische Zitterbewegungs beim Halten des Handstücks verursacht wird.

Der Zeitabstand kann alternativ auch mehr als 100 ms betragen. In diesem Fall wird die Aufnahme der 3D-Messdaten nachträglich mit der Aufnahme der Farbdaten anhand von Markierungen oder anhand von markanten Strukturen in beiden Aufnahmen überlagert.

### Kurzbeschreibung der Zeichnungen

Ausführungsbeispiele der Erfindung sind in der Zeichnung dargestellt. Es zeigt die
- Fig. 1: eine Skizze zur Verdeutlichung der Registrierung;
- Fig. 2: segmentierte erste Bereiche mit hartem Gewebe;
- Fig. 3: segmentierte zweite Bereiche mit weichem Gewebe;
- Fig. 4: eine Skizze einer alternativen Zahnsituation;
- Fig. 5: eine Skizze zur Verdeutlichung einer alternativen Gewichtung.

### Ausführungsbeispiele

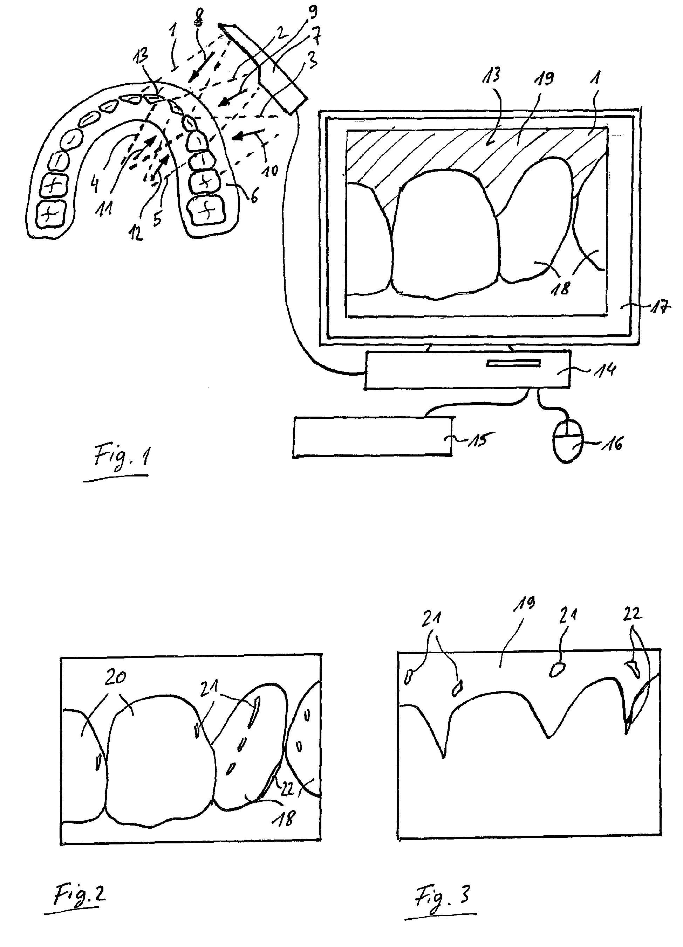

Die Fig. 1 zeigt eine Skizze zur Verdeutlichung des vorliegenden Verfahrens zur Registrierung mehrerer dreidimensionaler Aufnahmen, die durch gestrichelte Linien 1, 2, 3, 4, 5 angedeutet sind, eines dentalen Objekts 6, im vorliegenden Fall eines Oberkiefers. Die einzelnen Aufnahmen 1, 2, 3, 4 und 5 werden mittels des dentalen Handstücks 7 aus unterschiedlichen Aufnahmerichtungen 8, 9, 10, 11 und 12 aufgenommen, die durch Pfeile dargestellt sind. Das dentale Handstück umfasst erste Mittel zur dreidimensionalen Vermessung und zweite Mittel zur Farbmessung. Somit sind in jeder der Aufnahmen 3D-Messdaten und Farbdaten einer aufzunehmenden Messoberfläche 13 des Objekts 6 enthalten. Die Messdaten jeder Aufnahme werden vom dentalen Handstück 7 an einen Computer 14 übermittelt, an Bedienungsmittel, wie eine Tastatur 15 und eine Maus 16 angeschlossen ist, und mit einer Anzeigevorrichtung 17, wie mit einen Monitor, verbunden ist. In einem ersten Schritt kann ein Blur-Filter zur Glättung der 3D-Messdaten der Messoberfläche 13 auf die Aufnahme 1 angewendet werden. Zusätzlich kann ein weiterer Blur-Filter auf die Farbdaten angewendet werden, um die Farbdaten zu glätten und damit die Farbsegmentierung zu verbessern. Anschließend wird ein Segmentierungsverfahren durchgeführt, wobei erste Bereiche 18 mit hartem Gewebe, nämlich mit Zahnsubstanz und zweite Bereiche 19 mit weichem Gewebe, die Zahnfleisch abbilden, identifiziert. Diese beiden Schritte erfolgen für jede der Aufnahmen 1, 2, 3, 4 und 5. Anschließend werden die Aufnahmen 1, 2, 3, 4 und 5 unter Verwendung des computergestützten Registrierungsalgorithmus zu einer Gesamtaufnahme zusammengefügt. Dabei werden in den Aufnahmen 1, 2, 3, 4 und 5 übereinstimmende Strukturen mittels eines Strukturerkennungsalgorithmus erkannt und überlagert. Bei der Anwendung des Registrierungsalgorithmus werden die ersten Bereich 18 mit hartem Gewebe und die zweiten Bereiche 19 mit weichem Gewebe anhängig von der jeweiligen Situation mit unterschiedlichen Gewichtungsfaktoren gewichtet. Dadurch wird die Qualität der Registrierung verbessert.

Die Fig. 2 zeigt eine Skizze der durch die Verwendung des Segmentierungsverfahrens segmentierten ersten Bereiche 18 mit hartem Gewebe, die beispielsweise Zahnsubstanz abbilden. Die ersten Bereiche können auch Zahnersatz 20 abbilden, der in seiner Farbe erster Zahnsubstanz entspricht. Vor der Anwendung des Registrierungsalgorithmus werden zu helle Bereiche 21, die aufgrund von Glanzlicht entstehen, und zu dunkle Bereiche 22, die aufgrund einer unzureichenden Beleuchtung entstehen, aussortiert. Dadurch wird eine fehlerhafte Registrierung verhindert, denn die glänzenden Bereiche 21 und die schattigen Bereiche 22 sind abhängig von der Beleuchtung des dentalen Handstücks 7 und abhängig von der Aufnahmerichtung 8, 9, 10, 11 oder 12 in jeder der Aufnahmen 1, 2, 3, 4 oder 5 an unterschiedlichen Stellen.

Die Fig. 3 zeigt eine Skizze der zweiten Bereiche 19 mit weichem Gewebe, die entweder echtes Zahnfleisch oder Zahnfleischersatz abbilden können. Die zweiten Bereiche 19 wurden unter Verwendung des Segmentierungsverfahrens identifiziert. Vor der Durchführung der Registrierung werden wie in Fig. 2 zu helle Bereiche 21, die aufgrund von Glanzlicht entstehen und schattige Bereiche 22, die aufgrund unzureichender Beleuchtung entstehen, aussortiert. Dadurch wird, wie zu Fig. 2 erläutert, eine fehlerhafte Registrierung verhindert.

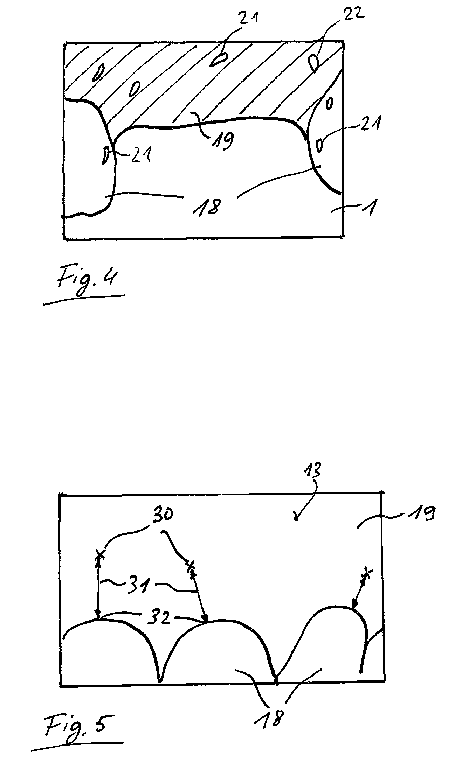

Die Fig. 4 zeigt eine Skizze einer anderen Zahnsituation als in Fig. 1. In Fig. 1 liegt ein erster Flächenanteil der ersten Bereiche 18 mit hartem Gewebe bei etwa 50 %. Ein zweiter Flächenanteil der zweiten Bereiche 19 mit weichem Gewebe liegt in Fig. 1 bei 30 % der Gesamtfläche der Aufnahme 1. Der restliche dritte Flächenanteil von dritten Bereichen, die keinem der Gewebearten zugeordnet werden können, liegt bei etwa 20 %. Der erste Flächenanteil liegt oberhalb eines festgelegt Schwellenwerts von 30%. Bei der vorliegenden Zahnsituation aus Fig. 1 wird daher der Schwellenwert überschritten, so dass die ersten Bereiche 18 mit hartem Gewebe bei der Anwendung des Registrierungsalgorithmus mit hoher Gewichtung berücksichtigt werden. Die zweiten Bereiche 19 mit weichem Gewebe werden mit geringer Gewichtung berücksichtigt oder gänzlich weggelassen.

Bei der Zahnsituation aus Fig. 4 fehlen im Vergleich zur Zahnsituation aus Fig. 1 die beiden mittleren Zähne, so dass der ersten Flächenanteil der ersten Bereiche 18 mit hartem Gewebe etwa 10 % der Gesamtfläche der Aufnahme 1 und die zweiten Bereiche 19 mit weichem Gewebe etwa 50 % der Gesamtfläche der Aufnahme 1 betragen. Der restliche dritte Flächenbereich beträgt etwa 40 %. Der festgelegte Schwellenwert für den ersten Flächenanteil der ersten Bereiche mit hartem Gewebe von 30% wird somit unterschritten. Folglich werden die ersten Bereiche mit einem ersten Gewichtungsfaktor und die zweiten Bereiche mit einem zweiten Gewichtungsfaktor gewichtet, wobei der zweite Gewichtungsfaktor bei einem abnehmenden ersten Flächenanteil zunimmt. Beispielsweise kann der erste Gewichtungsfaktor für die ersten Bereiche 18 1,0 betragen und der zweite variable Gewichtungsfaktor für die zweiten Bereiche 19 kann bei Überschreitung des Schwellenwerts 0,5 betragen und bis 1,0 bei abnehmendem ersten Flächenquotient ansteigen. Die Abhängigkeit des zweiten Gewichtungsfaktors vom Flächenquotient kann nach einer beliebigen Funktion, wie einer exponentialen Funktion oder einer linearen Funktion, festgelegt sein.

In Fig. 5 ist eine Skizze zur Verdeutlichung einer alternativen Gewichtung der ersten Bereiche 18 mit hartem Gewebe und der zweiten Bereiche 19 mit weichem Gewebe dargestellt. Dabei werden die ersten Bereiche 18 mit einem festen ersten Gewichtungsfaktor von beispielsweise 1,0 gewichtet. Die zweiten Bereiche 19 werden mit einem variablen zweiten Gewichtungsfaktor gewichtet, der für jeden Messpunkt 30 der Messoberfläche 13 bestimmt wird und bei einem zunehmenden Abstand 31 des jeweiligen Messpunkts zum nächstliegenden ersten Bereich 32 mit hartem Gewebe abnimmt. Die Abnahme des zweiten Gewichtungsfaktors in Abhängigkeit vom Abstand 31 zum nächstliegenden ersten Bereich 32 kann einer beliebigen Funktion, wie einer exponentialen Funktion oder einer linearen Funktion, erfolgen. Der zweite Gewichtungsfaktor beträgt an der Grenze zum nächstliegenden ersten Bereich 32 1,0 und fällt mit zunehmendem Abstand 31 bis zu einem Wert von 0. Damit wird das weiche Gewebe in einem kleinen Abstand zum nächstliegenden Bereich 32 mit hartem Gewebe gleich stark wie das harte Gewebe gewichtet. Dieses Verfahren führt zu einer Verbesserung der Registrierung, da das stärker berücksichtigte weiche Gewebe nahe am Zahn liegt und damit mit höherer Wahrscheinlichkeit fester und unbeweglicher ist.

### Bezugszeichenliste

- 1: gestrichelte Linie
- 2: gestrichelte Linie
- 3: gestrichelte Linie
- 4: gestrichelte Linie
- 5: gestrichelte Linie
- 6: Objekt
- 7: Handstück
- 8: Aufnahmerichtung
- 9: Aufnahmerichtung
- 10: Aufnahmerichtung
- 11: Aufnahmerichtung
- 12: Aufnahmerichtung
- 13: Messoberfläche
- 14: Computer
- 15: Tastatur
- 16: Maus
- 17: Anzeigevorrichtung/Monitor
- 18: erste Bereiche
- 19: zweiter Bereiche
- 20: Zahnersatz
- 21: heller Bereich
- 22: dunkler Bereich
- 30: Messpunkt
- 31: Abstand
- 32: nächstliegender erster Bereich

## Patentansprüche

1. Verfahren zur Registrierung mehrerer dreidimensionaler Aufnahmen eines dentalen Objekts (6), wobei jede der dreidimensionalen Aufnahmen (1, 2, 3, 4, 5) 3D-Messdaten und Farbdaten einer Messoberfläche (13) des Objekts (6) umfasst, wobei unter Verwendung eines computergestützten Registrierungsalgorithmus die einzelnen Aufnahmen (1, 2, 3, 4, 5) zu einer Gesamtaufnahme zusammengesetzt werden, wobei in jeder der Aufnahmen (1, 2, 3, 4, 5) unter Verwendung eines Segmentierungsverfahrens in Abhängigkeit von den Farbdaten erste Bereiche (18) mit hartem Gewebe und zweite Bereiche (19) mit weichem Gewebe identifiziert werden, wobei bei der Anwendung des Registrierungsalgorithmus die ersten Bereiche (18) und die zweiten Bereiche (19) mit unterschiedlichen Gewichtungsfaktoren gewichtet werden, **dadurch gekennzeichnet, dass** entweder die ersten Bereiche (18) vorteilshaft mit einem ersten Gewichtungsfaktor von 1,0 gewichtet werden und die zweiten Bereiche (19) mit einem zweiten variablen Gewichtungsfaktor gewichtet werden, der für jeden Messpunkt (30) der Messoberfläche bestimmt wird und bei einem zunehmenden Abstand (31) des Messpunktes zum nächstliegenden ersten Bereich mit hartem Gewebe abnimmt, oder
dass ein erster Flächenanteil der ersten Bereiche (18) und ein zweiter Flächenanteil der zweiten Bereiche (19) in jeder der Aufnahme bezogen auf die Gesamtfläche der jeweiligen Aufnahme berechnet wird, wobei falls der erste Flächenanteil der ersten Bereiche (18) einen Schwellenwert unterschreitet die ersten Bereiche mit einem ersten Gewichtungsfaktor und die zweiten Bereiche (19) mit einem zweiten Gewichtungsfaktor gewichtet werden, wobei der zweite Gewichtungsfaktor bei einem abnehmenden ersten Flächenanteil zunimmt.

2. Verfahren nach Anspruch 1, **dadurch gekennzeichnet, dass** die ersten Bereiche (18) Zahnsubstanz oder Zahnersatz (20) und die zweiten Bereiche (19) Zahnfleisch oder Zahnfleischersatz abbilden.

3. Verfahren nach Anspruch 1, **dadurch gekennzeichnet, dass** der Schwellenwert zwischen einem Wert von zwischen 5% und 30% festaeleat ist.

4. Verfahren nach Anspruch 1, **dadurch gekennzeichnet, dass** der zweite Gewichtsfaktor an der Grenze zum ersten Bereich einen Wert von 1,0 beträgt und mit zunehmendem Abstand (31) zum nächstliegenden ersten Bereich (18) bis zu einem minimalen Wert von 0,0 abnimmt.

5. Verfahren nach einem der Ansprüche 1 bis 4, **dadurch gekennzeichnet, dass** mithilfe von Schwellenwertkriterien, die auf die Farbdaten angewendet werden, dritte Bereiche (21, 22) identifiziert werden, die weder den ersten Bereichen (18) noch den zweiten Bereiche (19) mit weichem Gewebe zugeordnet werden, wobei die dritten Bereiche helle Bereiche (21), die aufgrund von Glanzlicht entstehen, und dunkle Bereiche (22), die aufgrund einer unzureichenden Beleuchtung entstehen, umfassen, wobei die Bereiche (21, 22) bei der Registrierung nicht berücksichtigt werden.

6. Verfahren nach einem der Ansprüche 1 bis 5, **dadurch gekennzeichnet, dass** vor der Segmentierung in erste Bereiche (18) und zweite Bereiche (19) ein Blur-Filter auf die Farbdaten in jede der Aufnahmen (1, 2, 3, 4, 5) angewendet wird.

7. Verfahren nach einem der Ansprüche 1 bis 6, **dadurch gekennzeichnet, dass** jede Aufnahme (1, 2, 3, 4, 5) mittels eines dentalen Handstücks (7) aufgenommen wird, das erste Mittel zur Erzeugung der 3D-Messdaten und zweite Mittel zur Erzeugung der Farbdaten umfasst, wobei die 3D-Messdaten und die Farbdaten gleichzeitig oder in einem kurzen Zeitabstand aus der gleichen Aufnahmerichtung (8, 9, 10, 11, 12) erfasst werden.

8. Verfahren nach Anspruch 7, **dadurch gekennzeichnet, dass** die 3D-Messdaten unter Verwendung der ersten Mittel, die auf einem Triangulationsverfahrens und einem Streifenprojektionsverfahrens beruhen, und die Farbdaten unter Verwendung einer herkömmlichen Farbkamera erzeugt werden.

9. Verfahren nach Anspruch 7, **dadurch gekennzeichnet, dass** der Zeitabstand zwischen 0,2 ms und 100 ms beträgt.

## Claims

1. A method for recording multiple three-dimensional images of a dental object (6), wherein each of the three-dimensional images (1, 2, 3, 4, 5) comprises 3D measured data and color data of a measured surface (13) of the object (6), wherein the individual images (1, 2, 3, 4, 5) are combined to form an overall image using a computer-assisted recording algorithm, wherein first regions (18) with hard tissue and second regions (19) with soft tissue are identified in each of the images (1, 2, 3, 4, 5) using a segmentation method, which depends on color data, wherein when using the recording algorithm, the first regions (18) and the second regions (19) are weighted with different weighting factors, **characterized in that** either the first regions (18) are weighted with a first weighting factor of 1.0, and the second regions (19) are weighted with a second variable weighting factor, which is determined for each measured point (30) of the measured surface and decreases as the distance (31) of the measured point from the most proximal first region with hard tissue increases or a first area section of the first regions (18) and the second area section of the second regions (19) in each of the images are calculated, based on the total area of the respective image, wherein the first regions are weighted with a first weighting factor when the first area section of the first regions (18) falls below a threshold value, and the second regions (19) are weighted with a second weighting factor, wherein the second weighting factor increases as the first area section decreases.

2. The method according to claim 1, **characterized in that** the first regions (18) depict tooth substance or a dental restoration (20), and the second regions (19) depict gingiva or gingiva substitute.

3. The method according to claim 1, **characterized in that** the threshold value is set between a value of between 5% and 30%.

4. The method according to claim 1, **characterized in that** the second weighting factor has a value of 1.0 at the border to the first region and decreases to a minimal value of 0.0 as the distance (31) from the most proximal first region (18) increases.

5. The method according to one of claims 1 through 4, **characterized in that** with the help of threshold value criteria, which are applied to the color data, third regions (21, 22), which are not assigned either to the first regions (18) or to the second regions (19) with soft tissue, are identified, wherein the third regions comprise light regions (21), which occur due to glaring light, and dark regions (22), which occur due to inadequate illumination, wherein the regions (21, 22) are not taken into account in the recording.

6. The method according to one of claims 1 to 5, **characterized in that** a blur filter is applied to the color data in each of the images (1, 2, 3, 4, 5) before the segmentation into first regions (18) and second regions (19).

7. The method according to one of claims 1 to 6, **characterized in that** each of the images (1, 2, 3, 4, 5) is recorded by means of a dental handpiece (7), comprising first means for generating 3D measured data and second means for generating the color data, wherein the 3D measured data and the color data are detected simultaneously or in a short interval of time from the same imaging direction (8, 9, 10, 11, 12).

8. The method according to claim 7, **characterized in that** the 3D measured data is generated using the first means which are based on a triangulation method and a strip projection method, and the color data is generated using a traditional color camera.

9. The method according to claim 7, **characterized in that** the interval of time is between 0.2 ms and 100 ms.

## Revendications

1. Procédé d'enregistrement de multiples enregistrements tridimensionnels d'un objet dentaire (6), où chacun des enregistrements tridimensionnels (1, 2, 3, 4, 5) comprend des données de mesurage 3D et des données de couleurs d'une surface de mesure (13) de l'objet (6), où l'utilisation d'un algorithme d'enregistrement informatisé permet de regrouper les enregistrements individuels (1, 2, 3, 4, 5) en un enregistrement intégral, où dans chacun de ces enregistrements (1, 2, 3, 4, 5), l'utilisation d'un procédé de segmentation, selon les données de couleurs, permet d'identifier les premières zones (18) avec un tissu dur et les deuxièmes zones (19) avec un tissu mou, où l'utilisation de l'algorithme d'enregistrement permet de pondérer les premières zones (18) et les deuxièmes zones (19) avec différents facteurs de pondération, **caractérisé en ce que** les premières zones (18) sont avantageusement pondérées avec un premier facteur de pondération 1,0 et les deuxièmes zones (19) avec un deuxième facteur de pondération variable, déterminé pour chaque point de mesurage (30) de la surface de mesure et qui, pour un écart croissant (31) du point de mesurage par rapport à la première zone la plus proche avec un tissu dur, diminue ou **en ce qu'**une première partie de la surface des premières zones (18) et une deuxième partie de la surface des deuxièmes zones (19) dans chaque enregistrement par rapport à la surface totale de l'enregistrement respectif sont calculées, où si la première partie de la surface des premières zones (18) est inférieure à une valeur seuil, ces premières zones sont pondérées avec un premier facteur de pondération et les deuxièmes zones (19) avec un deuxième facteur de pondération, où ce dernier augmente lorsque la première partie de la surface diminue.

2. Procédé selon la revendication 1, **caractérisé en ce que** les premières zones (18) représentent la substance de la dent ou la prothèse dentaire (20) et les deuxièmes zones (19) représentent la gencive ou l'implant.

3. Procédé selon la revendication 1, **caractérisé en ce que** la valeur seuil est définie entre une valeur comprise entre 5 % et 30 %.

4. Procédé selon la revendication 1, **caractérisé en ce que** la valeur du deuxième facteur de pondération à la limite de la première zone est 1,0 et diminue jusqu'à une valeur minimale de 0,0 lorsque la distance (31) à la première zone la plus proche (18) augmente.

5. Procédé selon l'une des revendications 1 à 4, **caractérisé en ce que** des critères de valeurs seuil appliqués aux données de couleurs permettent d'identifier des troisièmes zones (21, 22) qui ne sont affectées ni aux premières zones (18), ni aux deuxièmes zones (19) avec un tissu mou, où les troisièmes zones comprennent des zones claires (21) résultant du reflet et des zones sombres (22) résultant d'un éclairage insuffisant, où les zones (21, 22) ne sont pas prises en compte lors de l'enregistrement.

6. Procédé selon l'une des revendications 1 à 5, **caractérisé en ce que**, avant la segmentation en les premières zones (18) et les deuxièmes zones (19), un filtre flou est appliqué aux données de couleurs dans chacun des enregistrements (1, 2, 3, 4, 5).

7. Procédé selon l'une des revendications 1 à 6, **caractérisé en ce que** chaque enregistrement (1, 2, 3, 4, 5) est effectué à l'aide d'une pièce à main dentaire (7), comprenant un premier moyen de création de données de mesurage 3D et un deuxième moyen de création de données de couleurs, où les données de mesurage 3D et les données de couleurs sont saisies simultanément ou dans un intervalle de temps court, à partir de la même direction de prise de vues (8, 9, 10, 11, 12).

8. Procédé selon la revendication 7, **caractérisé en ce que** les données de mesurage 3D sont générées en utilisant le premier moyen reposant sur une méthode de triangulation et une technique de projection de franges et les données de couleurs sont générées en utilisant une caméra couleur traditionnelle.

9. Procédé selon la revendication 7, **caractérisé en ce que** l'intervalle de temps est compris entre 0,2 ms et 100 ms.
